Europäisches Patentamt

European Patent Office

Office européen des brevets

(11) Publication number: **0 573 920 A2**

# EUROPEAN PATENT APPLICATION

(21) Application number: 93109052.6

(22) Date of filing: 04.06.93

(51) Int. Cl.⁵: **C07C 17/26**, C07C 21/12

(30) Priority: **12.06.92 JP 179331/92**

(43) Date of publication of application:
**15.12.93 Bulletin 93/50**

(84) Designated Contracting States:
**DE FR**

(71) Applicant: **KUREHA CHEMICAL INDUSTRY CO., LTD.**
**9-11, Horidome-cho, 1-chome**
**Nihonbashi**
**Chuo-ku Tokyo 103(JP)**

(72) Inventor: **Yamaki, Shigetoshi**
**10-7, Sekita Shojigawa,**
**Nakosomachi**
**Iwaki-shi, Fukushima(JP)**
Inventor: **Monma, Nagahito**
**7-14, Nakaokamachi 4-chome**
**Iwaki-shi, Fukushima(JP)**
Inventor: **Miroku, Tomomichi**
**54, Shimonomae,**
**Ishizukamachi**
**Iwaki-shi, Fukushima(JP)**
Inventor: **Hoshino, Toshihiko**
**12-6, Chuo 3-chome,**
**Nishikimachi**
**Iwaki-shi, Fukushima(JP)**

(74) Representative: **Boeters, Hans Dietrich, Dr. et al**
**Patentanwälte Boeters Bauer Koepe**
**Bereiteranger 15**
**D-81541 München (DE)**

(54) Process for the manufacture of tetrachloroethylene.

(57) A process for the manufacture of tetrachloroethylene comprising steps of producing tetrachloroethylene by the reaction of a hydrocarbon having 3 or less carbon atoms, a partially chlorinated hydrocarbon having 3 or less carbon atoms, or a mixture of these, with carbon tetrachloride and chlorine gas in a reaction vessel at 500-700°C, obtaining the gas formed in the reaction at a state wherein the amount of carbon tetrachloride therein is equivalent to or smaller than the amount of the carbon tetrachloride supplied and the chlorine concentration therein is 7-15 mol%, separating and collecting tetrachloroethylene from said gas formed in the reaction by distillation, and recycling all amount of carbon tetrachloride in the gas formed in the reaction and chlorine gas to the reaction vessel for reuse. Recycling of carbon tetrachloride and chlorine gas ensures the conversion of the carbon tetrachloride into tetrachloroethylene in a closed system, prevents production of carbontetra chloride in the manufacturing of tetrachloroethylene, and makes it possible even to convert carbon tetrachloride by-produced in other processes into tetrachloroethylene.

EP 0 573 920 A2

## BACKGROUND OF THE INVENTION

### Field of the Invention

The present invention relates to a process for the manufacture of tetrachloroethylene.

### Description of the Prior Art

Tetrachloroethylene has conventionally been prepared by the thermal chlorinolysis of hydrocarbons having 3 or less carbon atoms, such as methane, ethane, ethylene, propane, and propylene, or their partial chlorinated products, with chlorine gas at a temperature of 450-750°C. The reaction accompanies production of carbon tetrachloride.

The production and the use of carbon tetrachloride in open systems are, however, supposed to be banned in the near future, because the carbon tetrachloride destroys the ozone layer. Carbon tetrachloride is not only produced together with tetrachloroethylene, but also produced as a by-product in the manufacture of chloroform from methane and in the manufacture of trichloroethylene from ethylene chloride.

Therefore, a number of trials have been undertaken to convert carbon tetrachloride into tetrachloroethylene. For example, a process for converting carbon tetrachloride into tetrachloroethylene by the disproportionation of carbon tetrachloride with methane or methylene chloride at a high temperature has been proposed (USP 3,726,932, Chem. Abstr. 79-18046k). The process, however, provides only a low selectivity of tetrachloroethylene. A process for thermally cracking carbon tetrachloride in the presence of nitrogen gas (Chem. Abstr. 59-178187t) only achieves a tetrachloroethylene yield of as low as 50% at a reaction temperature of 650-900°C. Although the yield is increased by the addition of chloroform, methylene chloride, hydrogen, methane, or the like, it involves the production of a tar-like material as a byproduct. A method for thermally cracking carbon tetrachloride in a quartz filled reactor only provides a tetrachloroethylene yield of as much as 77% (Chem. Abstr. 83-178187t). A method for thermally cracking carbon tetrachloride at 850-930°C in a carbon filled tube (Japanese Patent Laid-open (kokai) No. 117704/1975) involves side production of a large amount of hexachlorobenzene. In addition, the reaction requires a too high temperature for the method to be a practical one.

The equilibrium reaction of the following formula (1) is known between carbon tetrachloride and tetrachloroethylene (See, e.g., Journal of Industrial Chemistry, 70, (9), 1482-1485 (1967)).

$$2CCl_4 \rightleftharpoons C_2Cl_4 + 2Cl_2 \qquad (1)$$

The manufacture of tetrachloroethylene from carbon tetrachloride by the application of the equilibrium reaction of formula (1) is theoretically possible. However, since the formation of tetrachloroethylene is an endothermic reaction ($\Delta H_{298}$ = +185.5 kj) requiring a high temperature of about 700°C (Soda and Chlorine, 42, (8), 310 (1991)), it accompanies the production of a large amount of by-products with a high boiling point such as hexachlorobenzene. Thus, it is difficult to apply the method to the industrial manufacture of tetrachloroethylene.

Furthermore, a process for the manufacture of tetrachloroethylene from chlorides produced as by-products in the manufacture of vinyl chloride by adding ethylene to such chlorides and subjecting the mixture to the thermal chlorinolysis in the presence of circulated carbon tetrachloride under the conditions of a surplus amount of chlorine by 1 to 10 mol% has been proposed (Polish Patent No. 140824). This process also involves the production of high boiling point by-products such as partial chlorinated hydrocarbons, and thus is industrially disadvantageous.

In spite of the fact that the use of carbon tetrachloride will be banned, no industrially viable process which can convert the by-produced carbon tetrachloride into tetrachloroethylene has been developed. Therefore, development of a process for the manufacture of tetrachloroethylene which does not accompany the production of carbon tetrachloride has been desired.

## SUMMARY OF THE INVENTION

Accordingly, the object of the present invention is to provide a process for the manufacture of tetrachloroethylene which does not accompany the production of carbon tetrachloride and produces only a slight amount of highly chlorinated, high boiling point products which are difficult to convert into useful substances.

This object is achieved according to the present invention by a process for the manufacture of tetrachloroethylene comprising steps of,

producing tetrachloroethylene by the reaction of a hydrocarbon having 3 or less carbon atoms, a partially chlorinated hydrocarbon having 3 or less carbon atoms, or a mixture of these, supplied together with carbon tetrachloride, with chlorine gas at 500-700°C,

obtaining the gas formed in the reaction under a state wherein the amount of carbon tetrachloride therein is equivalent to or smaller than the amount of the carbon tetrachloride supplied and the chlorine concentration therein is 7-15 mol%,

separating and collecting tetrachloroethylene from said gas formed in the reaction by distillation, and

recycling all amount of carbon tetrachloride in the gas formed in the reaction and chlorine gas to the reaction vessel for reuse.

Other objects, features and advantages of the invention will hereinafter become more readily apparent from the following description.

## DETAILED DESCRIPTION OF THE INVENTION AND PREFERRED EMBODIMENTS

As hydrocarbons having 3 or less carbon atoms, propane, propylene, ethane, ethylene, methane, or a mixture of these hydrocarbons at any proportion can be used. Given as examples of partially chlorinated hydrocarbons having 3 or less carbon atoms are propylene chloride, ethylene chloride, trichloroethylene, trichloroethanes, dichloroethylenes, chloroform, methylene chloride, and the like, as well as partially chlorinated ethane or ethylene produced in the manufacture of vinyl chloride or vinylidene chloride. The partially chlorinated hydrocarbons in the present invention is thus chlorinated hydrocarbons, with not all the hydrogen atoms being substituted by chlorine, but leaving a part of hydrogen remained.

These hydrocarbons having 3 or less carbon atoms and/or partially chlorinated hydrocarbons having 3 or less carbon atoms (hereinafter referred to as "raw material hydrocarbons") are reacted with chlorine gas, while they are being supplied to the reaction vessel together with carbon tetrachloride, at a temperature of 500-700°C, preferably 550-650°C, and more preferably 560-630°C, and a reactor pressure of 100-200 kPa for 0.1-10 seconds, and preferably 0.5-5 seconds.

When the raw material hydrocarbons are fed together with carbon tetrachloride for the reaction with chlorine gas, as in the case of the process of the present invention, the raw material hydrocarbons produce tetrachloroethylene and carbon tetrachloride by the thermal chlorinolysis. At the same time, this reaction simultaneously accompanies a reaction in which carbon tetrachloride in an amount corresponding to the amount produced in the thermal chlorinolysis or more reacts with the raw material hydrocarbons to produce tetrachloroethylene.

When carbon tetrachloride is reacted with the raw material hydrocarbons or hydrogen, or a mixture of these, and converted into tetrachloroethylene in the reaction system where the thermal chlorinolysis of the raw material hydrocarbons proceeds, the amount of high boiling point materials such as hexachlorobenzene and the like produced is almost the same as the amount of these by-products produced in a simple thermal chlorinolysis of raw material hydrocarbons, thus enabling the carbon tetrachloride to be converted into tetrachloroethylene at a high yield.

Accordingly, the amount of carbon tetrachloride to be supplied to the above thermal chlorinolysis reaction of the raw material hydrocarbons should be the same as or greater than the amount produced in the thermal chlorinolysis. The ratio of the tetrachloroethylene and carbon tetrachloride in the gas composition (at the outlet of the reaction vessel) produced by the thermal chlorinolysis, as is well known, can be increased or decreased depending on the reaction conditions. The conditions are selected such that the amount of carbon tetrachloride in the reaction gas be equal to or smaller than the amount of the carbon tetrachloride supplied to the reactor. This makes it possible to recycle all amount of the produced carbon tetrachloride to the reaction vessel, taking tetrachloroethylene out of the system without the side production of carbon tetrachloride.

When the amount of carbon tetrachloride in the reaction gas is smaller than the amount of the carbon tetrachloride to be supplied, the thermal chlorinolysis of the raw material hydrocarbons is carried out while supplying carbon tetrachloride produced as a by-product in other reactions together with the carbon tetrachloride separated from the reaction gas of the thermal chlorinolysis of said raw material hydrocarbons. Besides producing tetrachloroethylene by the reaction with the raw material hydrocarbons, carbon tetrachloride produces tetrachloroethylene also by the reaction with hydrogen. Hydrogen, therefore, can be supplied as a part of the raw materials when an amount of carbon tetrachloride greater than the amount produced in the thermal chlorinolysis of the raw material hydrocarbons is to be converted into tetrachloroethylene.

The production of tetrachloroethylene according to the process of the present invention, taking propane as an example of the raw material, is governed by the following reactions.

$$C_3H_8 + 7Cl_2 \rightarrow 1.5C_2Cl_4 + 8HCl$$
$$C_3H_8 + 7CCl_4 \rightarrow 5C_2Cl_4 + 8HCl$$

In the operation of the process of the present invention, chlorine gas is supplied in excess of 11-25% of the amount required for the reaction with the hydrocarbons, so that the chlorine gas remains in the reaction gas in an amount of 7-15 mol%, and preferably 8-12 mol%. If the chlorine gas concentration in the reaction gas is smaller than 7 mol%, the side production of high boiling point products such as hexachlorobenzene increases. The concentration greater than 15 mol% is not desirable because of increased production of carbon tetrachloride according to the following formula.

$$C_2Cl_4 + 2Cl_2 \rightarrow 2CCl_4$$

When the relationship between the total amounts of carbon C (kg atom), hydrogen H (kg atom), and chlorine Cl (kg atom) satisfies the inequality, $2.5 < (Cl-H)/C < 3.5$, the chlorine concentration in the reaction gas is 7-15 mol%.

The reaction gas contains hydrogen chloride, tetrachloroethylene, and chlorine, as major components, and a small amount of high boiling point products such as hexachlorobenzene, hexachlorobutadiene, hexachloroethane, and the like. The concentration of the sum of tetrachloroethylene and carbon tetrachloride in the reaction gas should be controlled not to exceed 50 mol%, and preferably 40%. If this concentration is high, the production of high boiling point product increases. Such a concentration can be controlled by regulating the ratio of the total amount of carbon C (kg atom) and the total amount of hydrogen H (kg atom) to satisfy the inequality, $0.8 < H/C < 1.6$, or by introducing an appropriate amount of nitrogen gas into the reaction vessel.

The reaction gas is quenched through the contact with cold hydrochloric acid or cold water to liquefy and collect the components other than chlorine. The chlorine is recycled to the reactor after a dehydration treatment. The organic compounds collected by the liquefaction is separated from each other by distillation. The tetrachloroethylene fraction is purified into a high purity tetrachloroethylene product and the carbon tetrachloride fraction is all recycled to the reactor. The carbon tetrachloride fraction may contain a small amount of tetrachloroethylene, at most 10% or less, and preferably 5% or less. Increased inclusion of tetrachloroethylene in the recycled carbon tetrachloride fraction invites the increase in the side production of high boiling point products, and is thus undesirable.

The fraction consisting of hexachloroethane and hexachlorobutadiene is chlorinated under pressure in the presence of an iron chloride catalyst and converted into a mixture of hexachloroethane, octachlorobutene, and decachlorobutane to be recycled to the reactor, wherein they are converted into tetrachloroethylene. The hydrogen chloride is taken out as hydrochloric acid as a product, and the high boiling point products, of which a major component is hexachlorobenzene, are treated into innoxious products, usually by burning at a high temperature.

According to the process of the present invention, tetrachloroethylene can be produced without producing a substantial amount of carbon tetrachloride by effecting the thermal chlorinolysis and the reaction for tetrachloroethylene production from carbon tetrachloride and raw material hydrocarbons, while supplying the raw material hydrocarbons and chlorine together with carbon tetrachloride to the reaction system and controlling the chlorine concentration and the amount of carbon tetrachloride in the reaction gas.

Furthermore, according to the present invention, carbon tetrachloride produced in the other processes can be converted into tetrachloroethylene, enabling the tetrachloroethylene to be manufactured with the side production of high boiling point products in an amount as small as in the case where the reaction is limited to the thermal chlorinolysis.

Other features of the invention will become apparent in the course of the following description of the exemplary embodiments which are given for illustration of the invention and are not intended to be limiting thereof. In the examples hereinafter, "part(s)" for indicating amount of all materials means "part by weight per minutes", unless otherwise indicated.

EXAMPLES

Example 1

93.8 parts of propane, 250 parts of propylene chloride, 150 parts of a mixture having an average molecular formula of $C_2H_mCl_n$ (m = 2.39, n = 2.37; hereinafter the same), 61.9 parts of a mixture produced by chlorinating a by-product comprising hexachlorobutadiene as a major component under pressure, 2,394 parts of chlorine, and 1,519 parts of a carbon tetrachloride fraction (containing 5 wt.% of tetrachloroethylene; a pure carbon tetrachloride content: 1,443 parts) were introduced into a reaction vessel, which was operated under a pressure of about 105 kPa, a maximum temperature of 609°C, and a resident time of 2.2 seconds.

The composition of the reaction gas was hydrogen chloride 1,222 parts, carbon tetrachloride 1,443 parts, tetrachloroethylene 1,338 parts, chlorine 394 parts, hexachlorobenzene 23.8 parts, and other components 47.9 parts (a major component: hexachlorobutadiene; hereinafter the same), with the chlorine concentration of the reaction gas being 9.8 mol%.

The reaction gas was quenched by contacted with cold hydrochloric acid and then with cold water to collect a liquefied organic component mixture, which was distilled to separate each fraction, thus obtaining 1,263 parts of a tetrachloroethylene fraction, and 1,519 parts of a carbon tetrachloride fraction containing 5 wt.% of tetrachloroethylene. The carbon tetrachloride fraction and chlorine were recycled to the reactor, and 47.9 parts of the other components comprising hexachlorobutadiene as a major component was chlorinated under pressure (the amount after the chlorination under pressure: 61.9 parts) and recycled to the reactor.

The amount of hexachlorobenzene for 100 parts of the product tetrachloroethylene was 1.9 parts.

Example 2

102.5 parts of propane, 250 parts of propylene chloride, 150 parts of a mixture having an average molecular formula of $C_2H_mCl_n$, 68.4 parts of a mixture produced by chlorinating a by-product comprising hexachlorobutadiene as a major component under pressure, 2,389 parts of chlorine, 200 parts of carbon tetrachloride, and 1,438 parts of a carbon tetrachloride fraction (not containing tetrachloroethylene) were introduced into a reaction vessel, which was operated under a pressure of about 105 kPa, a maximum temperature of 609°C, and a resident time of 2 seconds.

The composition of the reaction gas was hydrogen chloride 1,280 parts, carbon tetrachloride 1,438 parts, tetrachloroethylene 1,409 parts, chlorine 389 parts, hexachlorobenzene 29.6 parts, and other components 52.3 parts, with the chlorine concentration of the reaction gas being 9.4 mol%.

The reaction gas was quenched by contacted with cold hydrochloric acid and then with cold water to collect a liquefied organic component mixture, which was distilled to separate each fraction, thus obtaining 1,409 parts of a tetrachloroethylene fraction, and 1,438 parts of a carbon tetrachloride fraction. The carbon tetrachloride fraction was recycled to the reactor, and 52.3 parts of the other components comprising hexachlorobutadiene as a major component was chlorinated under pressure (the amount after the chlorination under pressure: 68.4 parts) and recycled to the reactor.

The amount of hexachlorobenzene for 100 parts of the product tetrachloroethylene was 2.1 parts.

Comparative Example 1

109.3 parts of propane, 250 parts of propylene chloride, 150 parts of a mixture having an average molecular formula of $C_2H_mCl_n$, 111.4 parts of a mixture produced by chlorinating a by-product comprising hexachlorobutadiene as a major component under pressure, 2,274 parts of chlorine, and 1,350 parts of a carbon tetrachloride fraction (containing 20 wt.% of tetrachloroethylene) were introduced into a reaction vessel, which was operated under a pressure of about 106 kPa, a maximum temperature of 612°C, and a resident time of 2 seconds.

The composition of the reaction gas was hydrogen chloride 1,325 parts, carbon tetrachloride 1,080 parts, tetrachloroethylene 1,569 parts, chlorine 274 parts, hexachlorobenzene 114.9 parts, and other components 81.8 parts, with the chlorine concentration of the reaction gas being 6.7 mol%; less than 7 mol%.

The reaction gas was quenched by contacted with cold hydrochloric acid and then with cold water to collect a liquefied organic component mixture, which was distilled to separate each fraction, thus obtaining 1,299 parts of the product tetrachloroethylene. 1,350 parts of a carbon tetrachloride fraction (containing 20 wt.% of tetrachloroethylene) was recycled to the reactor, and 81.8 parts of the other components comprising hexachlorobutadiene as a major component was chlorinated under pressure (the amount after the chlorination under pressure: 111.4 parts) and recycled to the reactor.

The amount of hexachlorobenzene for 100 parts of the product tetrachloroethylene was 8.8 parts.

Comparative Example 2

97.8 parts of propane, 250 parts of propylene chloride, 150 parts of a mixture having an average molecular formula of $C_2H_mCl_n$, 79.4 parts of a mixture produced by chlorinating a by-product comprising hexachlorobutadiene as a major component under pressure, 2,238 parts of chlorine, and 200 parts of carbon tetrachloride, 928 parts of a carbon tetrachloride fraction (containing 5 wt.% of tetrachloroethylene; pure carbon tetrachloride content: 882 parts) were introduced into a reaction vessel, which was operated under a pressure of about 105 kPa, a maximum temperature of 609°C, and a resident time of 2 seconds.

The composition of the reaction gas was hydrogen chloride 1,249 parts, carbon tetrachloride 882 parts, tetrachloroethylene 1,238 parts, chlorine 238 parts, hexachlorobenzene 77.1 parts, and other components 59.1 parts, with the chlorine concentration of the reaction gas being 6.6 mol%; less than 7 mol%.

The reaction gas was quenched by contacted with cold hydrochloric acid and then with cold water to collect a liquefied organic component mixture, which was distilled to separate each fraction, thus obtaining 1,192 parts of tetrachloroethylene and 928 parts of a carbon tetrachloride fraction containing 5 wt.% of tetrachloroethylene. The carbon tetrachloride fraction was recycled to the reactor, and 59.1 parts of the other components comprising hexachlorobutadiene as a major component was chlorinated under pressure (the amount after the chlorination under pressure: 79.4 parts) and recycled to the reactor.

The amount of hexachlorobenzene for 100 parts of the product tetrachloroethylene was 6.5 parts.

Obviously, numerous modifications and variations of the present invention are possible in light of the above teachings. It is therefore to be understood that within the scope of the appended claims, the invention may be practiced otherwise than as specifically described herein.

## Claims

1.  A process for the manufacture of tetrachloroethylene comprising steps of,

    (a) producing tetrachloroethylene by the reaction of a hydrocarbon having 3 or less carbon atoms, a partially chlorinated hydrocarbon having 3 or less carbon atoms, or a mixture of these, supplied together with carbon tetrachloride, and chlorine gas in a reaction vessel at 500-700°C,

    (b) obtaining the gas formed in the reaction at a state wherein the amount of carbon tetrachloride therein is equivalent to or smaller than the amount of the carbon tetrachloride supplied and the chlorine concentration therein is 7-15 mol%,

    (c) separating and collecting tetrachloroethylene from said gas formed in the reaction by distillation, and

    (d) recycling all amount of carbon tetrachloride in the gas formed in the reaction and chlorine gas to the reaction vessel for reuse.

2.  A process according to Claim 1, wherein said reaction in said step (a) is carried out at a temperature of 550-650°C under a pressure of 100-200 kPa for 0.1-10 seconds.

3.  A process according to Claim 1, wherein the concentration of the sum of tetrachloroethylene and carbon tetrachloride in the reaction gas from said step (a) is controlled so as not to exceed 50 mol%.